## Europäisches Patentamt
## European Patent Office
## Office européen des brevets

(11) Publication number: **0 140 109**
**B1**

(12) # EUROPEAN PATENT SPECIFICATION

(45) Date of publication of patent specification: **23.01.91**

(21) Application number: **84110998.6**

(22) Date of filing: **14.09.84**

(51) Int. Cl.⁵: **A 61 K 39/00**, A 61 K 39/44, C 07 K 17/06

(54) Allograft of reduced immunogenicity and method and reagent for making same.

(30) Priority: **15.09.83 US 532609**

(43) Date of publication of application:
**08.05.85 Bulletin 85/19**

(45) Publication of the grant of the patent:
**23.01.91 Bulletin 91/04**

(84) Designated Contracting States:
**AT BE CH DE FR GB IT LI NL SE**

(56) References cited:
EP-A-0 080 401
GB-A-2 093 037
US-A-4 489 710

SCIENCE, vol. 222, no. 4623, 4th November 1983, pages 512-515; D.A. VALLERA et al.: "Anti-T-cell reagents for human bone marrow transplantation: ricin linked to three monoclonal antibodies"

(73) Proprietor: **THE BOARD OF TRUSTEES OF THE LELAND STANFORD JUNIOR UNIVERSITY**
**Encina 105 Stanford University**
**Stanford California 94305 (US)**

(73) Proprietor: **CETUS CORPORATION**
**1400 Fifty-Third Street**
**Emeryville California 94608 (US)**

(72) Inventor: **Fathman, C. Garrison**
**570 Oak Knoll Lane**
**Menlo Park California 94025 (US)**

(74) Representative: **Vossius & Partner**
**Siebertstrasse 4 P.O. Box 86 07 67**
**D-8000 München 86 (DE)**

Courier Press, Leamington Spa, England.

56 References cited:

CHEMICAL ABSTRACTS, vol. 96, no. 17, 26th
April 1982, page 593, abstract no. 140999q,
Columbus, Ohio, US; D.A. VALLERA et al.:
"Bone marrow transplantation across major
histocompatibility barriers. V. Protection of
mice from lethal graft-vs.-host desease by
pretreatment of donor cells with monoclonal
anti-Thy-1.2 coupled to the toxin ricin", & J. EXP.
MED. 1982, 155(3), 949-54

TISSUE ANTIGENS, vol. 16, 1980, pages 30-48,
Munksgaard, Copenhagen, DK; F.M. BRODSKY
et al.: "Monoclonal antibodies to HLA-DRw
determinants"

## Description

The invention is in the field of transplantation immunology and concerns a technique for reducing the immune response of a recipient individual against an allograft by treating the allograft before it is transplanted with an immunotoxin directed against an la equivalent antigen (e.g., an HLA-DR antigen) borne by the allograft.

Transplants between genetically dissimilar individuals of the same species (called allogeneic grafts or allografts) normally induce an immune response in the recipient or host individual. The immune response often leads to rejection or destruction of the allograft. This response is believed to be primarily a T cell mediated response to cell surface antigens that distinguish donor from recipient.

Various treatments of donors and/or recipients to enhance allograft survival have been reported. Recipients have been treated with immunosuppressing drugs such as antimitotic agents and adrenal steroids to reduce the ability of the recipient to respond to the allograft. This mode of treatment usually leaves the recipient immunodeficient and thus susceptible to infection. Various antisera have also been tested as allograft survival enhancers. Davies, D. A. L. and Staines, N. A., *Transplant Rev.* (1979) *30*: 18—39 report enhanced organ graft survival in rodents by passive immunization of recipients with anti-la antibodies. A number of references describe treating donors with anti-lymphocyte serum (ALS) to enhance allograft survival. In this regard Hornung, M. O., et al., *J. Immun.* (1971) *107*: 979—984, describe treating treating test leukocytes with anti-HLA-A $F(ab')_2$ to eliminate their ability to stimulate target leukocytes in a mixed lymphocyte reaction (MLR). The article postulates that the $F(ab')_2$ bound to HLA-A antigen on the test leukocytes and prevented them from stimulating the target lymphocytes. Another group of references report that allograft survival may be enhanced by removing passenger leukocytes from the allograft by *in vivo* culture of the allograft. *Surgery* (1977) *81*: 74—79; *Science* (1980) *209*: 183—185; and *Trans. Proc.* (1982) *8*: 1094—1098. Faustman, D., et al., *Transplantation* :1982) *34*: 302—305 report on the survival of heart allografts in nonimmunosuppressed murine recipients by pretreatment of donor tissue with anti-la antibodies.

Immunotoxins are conjugates of bacterial or plant toxins, such as diphtheria toxin or ricin, and antibodies. The field of immunotoxins has been reviewed recently by Olsnes, S. and Pihl, A., *Pharmac. Ther.* (1982) *15*: 335—381 and Jansen, F. K., et al., *Immun. Rev.* (1982) *62*: 185—216. Immunotoxins have been used primarily as antineoplastic agents, with the antibody portion of the conjugate being directed against target tumors. Applicant knows of no prior immunotoxin involving antibodies directed against HLA-DR antigens (or analogous antigens in other mammalian species). The only prior use of immunotoxins in transplantation of which applicant is aware is the use of antibody-ricin conjugates to eradicate malignant cells in autogeneic bone marrow transplants (Mason, D. W., et al., *Cancer Surveys* (1982) *Vol. 1, No. 3*: 389—415 and Vitetta, E. S., et al., *Science* (1983) *219*: 644—650) and to eradicate immunocompetent T lymphocytes in murine bone marrow to eliminate graft-vs-host disease (Vallera, D. A., et al., *J. Exp. Med.* (1982) *155*: 949—954).

The present invention was premised on applicant's conception that it might be possible to enhance allograft survival more effectively by depleting the allograft of cells that stimulate responder cells in the recipient by treating the allograft with an appropriate immunotoxin. Applicant tested this hypothesis and found it valid by treating rodent allografts with an anti-la immunotoxin to eradicate their stimulator cells.

Accordingly, one aspect of the invention is a method of reducing the immunogenicity of an allograft characterized in that the allograft is treated before transplantation with an immunotoxin to an la equivalent antigen borne by the allograft.

Another respect of the invention is an allograft characterized in that the allograft is pretreated (i.e., treated before transplantation) with an immunotoxin to an la equivalent antigen borne by the allograft.

A third aspect of the invention is an immunotoxin comprising a toxin moiety conjugated to an antibody characterized in that the immunotoxin is for use in reducing the immunogenicity of an allograft and the antibody is to an la equivalent antigen borne by the allograft.

In the drawings:

Figure 1 is a graph of the results of the MLRs described in Example 1, infra; and

Figure 2 is a graph of the results of the MLRs described in Example 2, infra.

As used herein the term "la equivalent antigen" is intended to denote a gene product of the major histocompatibility complex (MHC) that stimulates an immunological reaction in a given MLR. This term is intended to be nonspecies specific and thus include not only murine gene products such as those of the IA and IE regions but also functionally similar products of analogous MHC regions of other mammalian species such as the DR, SV or DC regions of the HLA. In the case of humans, the term will normally denote an HLA-DR antigen. It follows that an anti-la equivalent antibody is an antibody that reacts with an la equivalent antigen present on the stimulator cells in a given MLR.

The term "antibody" as used herein is intended to include polyclonal and monoclonal antibodies and antigen binding fragments (Fab, Fab', $F(ab')_2$ and Fv) thereof.

The term "allograft" is intended to denote a multiplicity of individual mammalian cells or a multiplicity of associated mammalian cells that define a tissue or organ from an individual that is genetically dissimilar to the intended recipient. It will usually refer to skin or an organ that does not have an la equivalent-bearing endothelium and whose parenchymal cells lack Ia equivalent antigens such as the endocrine glands (pituitary, thyroid, adrenal, parathyroid, and pancreas).

As used herein the term "immunogenicity" refers to the ability of the allograft to elicit an immune response when it is transplanted into a genetically dissimilar individual.

The cytotoxic portion of the anti-Ia equivalent immunotoxin may be a bacterial or plant toxin, a portion of such a toxin that includes its enzymatically active fragment, or a protein that exhibits enzymatic activity similar to the enzymatic activity of such a toxin. These toxins and proteins are polypeptides. Their enzymatic activity enables them to inhibit cellular protein synthesis, such as by inactivating elongation factor-2 or inactivating ribosomal 60s subunits, once they are internalized. Examples of such toxins and proteins are diphtheria toxin, exotoxin (from *Pseudomonas aeruginosa*), ricin, abrin, modeccin, alpha-sarcin, *Aleurites fordii* proteins, dianthin proteins, *Phytolacc americana* proteins (PAPI, PAPII, and PAP-S), momordin, curcin, crotin, gelonin, mitogellin, restrictocin, phenomycin, and enomycin. These toxins and proteins may be extracted or otherwise separated from appropriate bacteria or plants. Enzymatically active fragments of such toxins may be obtained by breaking the bonds between the active (A) chain of the toxin and the binding (B) chain(s) of the toxin (e.g., reducing the disulfide bond(s) between the A and B chain(s) with an appropriate reducing agent, such as 2-mercaptoethanol or dithiothreitol) and isolating the A chain from the B chain. It should also be possible to produce the similar acting proteins or at least the enzymatically active fragments of the toxins by current genetic engineering techniques.

The antibody components of the immunotoxins are obtained or prepared by conventional procedures. Anti-Ia equivalent sera may be obtained from immunized individuals known to have a high titer of the desired antibody. In the case of humans pregnancy sera or sera from individuals having a prior transplantation history are sources of anti-Ia equivalent antibody. Anti-Ia equivalent antibodies may be prepared by immunizing host animals, e.g., horse, goat, rabbit, sheep, with B cells from individuals of the appropriate Ia equivalent type and collecting serum from the immunized host.

Monoclonal anti-Ia equivalent antibodies may be made by the somatic cell hybridization procedure first described by Köhler, G. and Milstein, C., *Nature* (1975) *256*: 495—497. The tumor cell lines, reagents, and conditions used in this procedure are well known and have been reviewed extensively in the literature (*Somatic Cell Genetics*, (1979) *5*: 957—972). Briefly, the procedure involves immunizing a host with the immunogen of interest (B cells from an individual of appropriate Ia equivalent type), collecting antibody-producing cells from the immunized host, fusing the antibody-producing cells from the immunized host with an appropriate tumor cell line using a fusogen such as a polyethylene glycol, growing the cells in a selective medium to eliminate unhybridized partners, identifying hybridomas that produce antibody against the immunogen, growing such hybridomas, and collecting monoclonal antibody from the resulting culture medium (or body fluid when grown *in vivo*). Monoclonal antibodies of current interest will typically be of human, rat or murine origin since rat, mouse and human tumor cell lines are available for fusion. There are numerous reports of anti-HLA-DR monoclonal antibodies in the literature. See, for instance Grumet, F. C., et al., *Human Immunology* (1983) *6*: 63—73; and Palacios, R., et al., *Proc. Natl. Acad. Sci. U.S.A.* (1983) *80*: 3456—3460 and *Europ. J. Immunol.* (1983) *13*: 64—72. An anti-HLA-DR monoclonal antibody is currently being sold by Becton-Dickinson, Sunnyvale, California.

Antigen binding fragments (Fab, Fab', F(ab')$_2$, Fv) of polyclonal or monoclonal antibodies may be made by digesting the whole Ig with an appropriate protease, for instance papain in the case of Fab and pepsin in the case of F(ab')$_2$.

The class (and subclass) of the antibody used is not critical. When antiserum is used it is highly likely that a spectrum of Ig classes will be present that react with Ia equivalent antigen. When a monoclonal antibody is used it is most likely that the antibody will be an IgG since this class is predominant. Mixtures of monoclonals directed against the Ia equivalent antigen present on the stimulating B cells of the allograft may be used, if desired. Likewise, the species of antibody is not critical. As indicated above the antibody is likely to be of rodent or human origin because of the availability of suitable human and rodent tumor lines.

The toxin portion of the immunotoxin is conjugated covalently to the antibody using standard conjugation techniques and available bifunctional coupling agents if necessary. Olsnes, S. and Pihl, A., *supra*. If the toxin and antibody both contain free reactive amino acids (e.g., free —SH groups on cysteine residues), they may be conjugated without using coupling agents. Bifunctional disulfides such as 3,3'-dimethyldithio-bispropionate and N-succinimidyl-3-(2-pyridyldithio)propionate (SPDP) have been used to couple toxin and antibody via a reducible disulfide bridge. Examples of other coupling agents are the N-hydroxysuccinimido esters of 6-maleimidocaproic acid, 2-bromoacetic acid, and 2-iodoacetic acid, other active esters of such acids, imidoesters such as dimethyladipimidate, disuccinimidyl suberate, aldehydes such as glutaraldehyde, bis-azido compounds such as bis-(*p*-azidobenzoyl)hexanediamine, bis-diazonium derivative, and diisocyanates such as tolylene-2,6-diisocyanate, and carbodiimides.

The allograft is treated with the immunotoxin under conditions that permit the immunotoxin to abrogate at least a substantial portion, preferably all, of the Ia equivalent antigen-bearing cells of the allograft. Many human allografts are obtained from cadavers. Such allografts may be treated with the immunotoxin either after beign removed or *in situ*. Allografts obtained from living donors will be treated after being removed. When treated after being removed the allograft will either be perfused in vitro with a perfusion medium containing the immunotoxin, or where permitting, maintained in a culture medium containing the immunotoxin. The immunotoxin will usually be added to the perfusion or culture medium in amounts in excess of about 1 µg, usually 1 to 10 µg, immunotoxin per 1 × $10^6$ la equivalent antigen-bearing cells. MLR experiments indicates the immunotoxin will rapidly eradicate stimulator cells upon contact. The efficiency of the contact between the immunotoxin and the cells is, therefore, likely to determine the treatment duration. Since there will usually be no reason to carry out the treatment rapidly, the treatment duration will usually be at least about 15 min, or longer when the allograft is large. Immunotoxins are typically water-soluble and may be formulated in aqueous media commonly used for parenteral administration of drugs. When the allograft is treated *in situ* the cadaver containing it may be perfused intravenously with the immunotoxin. In the case of nonhuman allografts the host may be perfused *in vivo* with the immunotoxin and sacrificed to obtain the transplant. Intermittent or continuous treatment *in vivo* may be desirable if the immunotoxin has a relatively short half-life.

The effectiveness of the invention in reducing the immunogenicity of the donor cells may be assayed *in vitro* by subjecting samples of the treated allograft to an MLR test in which a small sample of the treated allograft is mixed with lymphocytes from the recipient in a cell culture medium.

The treated allograft may be introduced to the recipient by conventional means. For instance, in skin grafting the treated skin is placed and held at the graft site until the graft takes to the subdermal tissue. Treated organs are introduced surgically. Allografts that are to be introduced into the host for therapeutic or diagnostic purposes will typically be administered in combination with a therapeutically acceptable vehicle or carrier by injection. As used herein the term "transplanting" is intended to encompass all such methods of introduction.

The following examples illustrate the immunotoxins, allograft tissue treated with them, and the effectiveness of the treatment in reducing allograft immunogenicity. These examples are not intended to limit the invention in any way.

## Example 1
### In vitro Treatment

Preparation of Immunotoxin

An anti-la monoclonal antibody designated 13.4 was obtained from Dr. Günter Hämmerling, Heidelberg, Federal Republic of Germany. This antibody is described in *Immunogenet* (1979) *8*: 433—445. It reacts with the I—$E^k$ molecules and is associated with la7 specificity. This antibody cross reacts with rat class II MHC antigens. Monoclonal antibody 13.4 (2 mg/ml in phosphate buffered saline, PBS) was derivatized with the heterobifunctional cross-linking reagent N-succinimidyl-3-(2-pyridyldithio) propionate (SPDP; Pharmacia, Piscatway, New Jersey). SPDP (0.075 ml, 20 mM) in absolute ethanol was added to each ml of antibody solution, followed by incubation at room temperature for 30 min. The derivatized antibody was extensively dialyzed against PBS at 4°C to remove residual unreacted SPDP, then analyzed for pyridine-2-disulfide content as described by Carlson, et al., *Biochem. J.* (1978) *173*: 723—737. An average of 4 such residues was found per molecule of antibody.

Ricin A chain (RTA) was purchased from EY Labs, San Mateo, California. This RTA was essentially nontoxic to a variety of cultured cell lines, requiring a dose of 0.1 µM to inhibit protein synthesis by 50%. RTA (0.8 mg/mL) was freshly reduced by adding dithiothreitol (1 M, pH 7.0) to a final concentration of 50 mM. Following incubation for 30 min. at room temperature, the reduced RTA was desalted on a G—25 column equilibrated in PBS. Those fractions containing the highest protein concentrations were pooled. The reduced, desalted RETA was then mixed with derivatized antibody. Final concentrations of derivatized antibody and RTA were 4.4 µM and 10 µM, respectively.

The conjugate mixture herein designated 13.4-RTA was incubated at room temperature for one hr., then stored at 4°C. Successful conjugation was demonstrated by sodium dodecyl sulfate polyacrylamide gel electrophoresis (SDS-PAGE) and by spectrophotometric determination of the quantity of pyridine-2-thione released upon mixing the derivatized antibody with RTA. An average of 1.5 molecules of RTA were covalently coupled to each molecule of antibody. Since the RTA used for these experiments was essentially nontoxic, residual unreactive RTA was not removed from the conjugation mixture.

Mixed Lymphocyte Reactions (MLR)

*Cells*

Male ACI rats, 11—18 weeks of age and 200—280 grams of weight, were obtained from Simonsen Laboratories, Gilroy, California. Male Lewis x Brown Norway (LBN) $F_1$ rats, 12—18 weeks of age and 300—400 grams of weight, were obtained from Harlan Sprague-Dawley Company, Walkersville, Maryland.

Spleen and lymph node cells taken from the animals were teased in RPMI 1640 (Gibco, Grand Island, New York) supplemented with 10% fetal calf serum (M.A. Bioproducts, Walkersville, Maryland), 40 µm 2-mercaptoethanol (J. T. Baker Chemical Co., Phillipsburg, New Jersey), 2 mM L-glutamine (Gibco), 12 mM Hepes (Gibco), 100 U/mL penicillin + 100 µg/ml streptomycin (Gibco). Cell suspensions were centrifuged at 1,200 rpm for 10 min and washed twice.

*Methods*

MLRs were carried out using stimulator cells treated with 13.4-RTA. For comparison purposes MLRs were also carried out using 13.4 and 13.4 plus rabbit complement. Untreated syngeneic and untreated allogeneic MLRs were run as controls. Details of the procedures used are given below.

*13.4 Treatment.* Responder (ACI) lymph node cells, $0.5 \times 10^6/0.1$ mL of irradiated (3,300 rads) stimulator (LBN) spleen cells in a well of a Falcon 3072 microtiter plate (Becton Dickinson) and serial concentrations of monoclonal antibody 13.4 (0.001, 0.01, 0.1, 1 µg per $1 \times 10^6$ stimulator cells) were added in each well to block MLR response. Cultures were incubated at 37°C in a humidified atmosphere at 5% $CO_2$/ 95% air for four days, labeled with tritiated thymidine (1 µCi/well) and incubated for a further 18 hr. Cells were harvested onto glass filters (M.A. Bioproducts) and thymidine incorporation was determined by liquid scintillation counting (Beckman LS 6800, Beckman Instruments, Inc.).

*13.4 Plus Complement Treatment.* Stimulator spleen cells were pretreated with serial concentrations of the 13.4 (0.001, 0.01, 1 µg per $1 \times 10^6$ stimulator cells) for 30 min. at 4°C, washed twice and incubated with 1:20 dilute rabbit complement for 30 min. at 37°C. They were irradiated (3,300 rads) after washing twice, adjusted to $1 \times 10^6$ viable cells with fluorescent diacetate (FDA) in 0.1 ml culture media and cultured with $0.5 \times 10^6/0.1$ mL lymph node cells as responder for four days. Thymidine incorporation was assayed as above.

*13.4-RTA Treatment.* Stimulator spleen cells were centrifuged at 1,200 rpm for 10 min. Lactose, 100 mM (to inactivate whole ricin contaminant in the 13.4-RTA) and serial concentrations of the 13.4-RTA (0.001, 0.01, 0.1, 1 µg antibody per $1 \times 10^6$ stimulator cells) were added to the pellet of stimulator cells and incubated for 60 min. at 37°C. $1 \times 10^6$ Viable irradiated 13.4-RTA-treated spleen cells ($1 \times 10^6$) in 0.1 ml culture medium were cultured with $0.5 \times 10^6$ lymph node cells for four days and thymidine incorporation was determined as above.

The percent suppression (%S) achieved by these treatments was calculated with the following formula:

$$\%S = \left( \frac{1\text{-cpm of experimental MLR-cpm of untreated syngeneic MLR}}{\text{cpm of untreated allogeneic MLR-cpm of untreated syngeneic MLR}} \right) \times 100$$

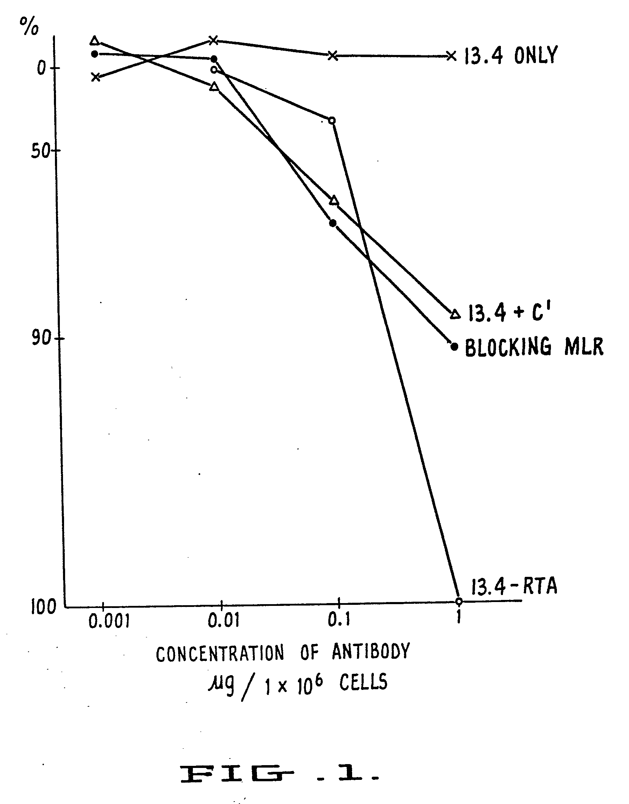

The results of these tests are shown in Figure 1. %S is plotted against antibody concentration. As shown at a concentration of 1 µg/$1 \times 10^6$ stimulator cells the 13.4-RTA provided 100% suppression and was more effective than either of the comparison treatments. The reciprocal MLR using ACI cells as the stimulator and LBN cells as the responder was carried out with almost identical results. Similar results were also obtained using stimulator cells incubated with 13.4-RTA for as little as five min. at 4°C.

## Example 2
### In vivo Treatment

A/J ((A, $H-2^k$ and C57B1/6 (B6, $H-2^b$) mice were obtained from Jackson Laboratories, Bar Harbor, Maine. Adult male mice, aged 12—18 weeks, were used.

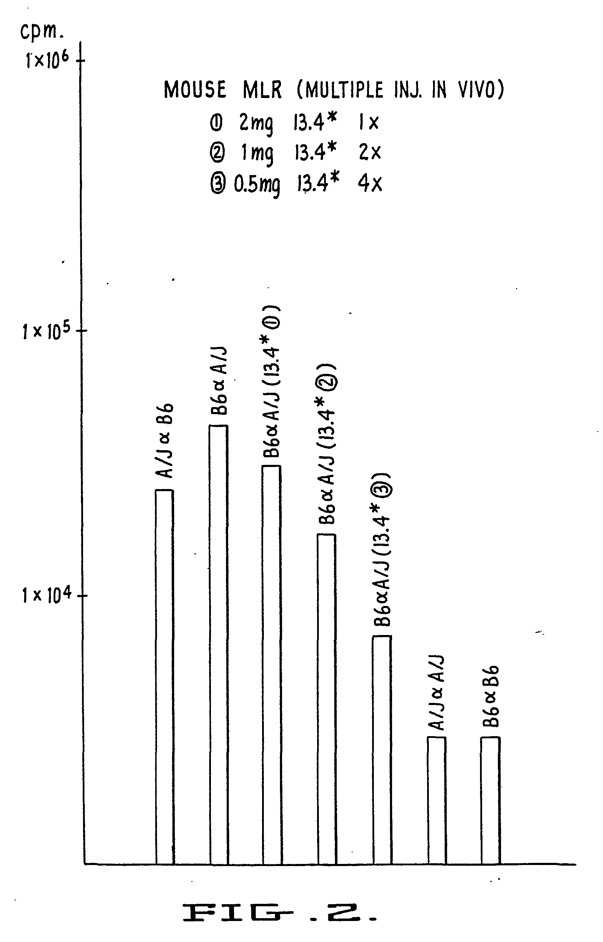

Strain A/J mice were given multiple IV injections of 13.4-RTA according to the following regimens: 2 mg 1×; 1 mg 2× (every one hr.); and 0.5 mg 4× (every one hr.). Four hr. after the initial injection spleen cells were taken from the animals and treated as described under the subheading "Cells" in Example 1. MLRs were carried out using these cells as described under the subheading "13.4-RTA Treatment" in Example 1. Control MLRs were run using A/J spleen cells from mice that had not been treated with the conjugate. Figure 2 is a bar graph showing the thymidine incorporation (expressed as cpm) observed in these MLRs. As shown, the MLR was substantially suppressed by the *in vivo* treatment of the A/J stimulator cells with 13.4-RTA. The increased efficacy observed with increasing numbers of injections is believed to be an indication that the conjugate is labile *in vivo*.

Modifications of the modes for carrying out the invention that are described above that are obvious to those of skill in medicine, immunology, and/or molecular biology are intended to be within the scope of the following claims.

## Claims

1. Method of reducing the immunogenicity of an allograft characterised in that the allograft is contacted in vitro before transplantation with an immunotoxin to an Ia equivalent antigen borne by the allograft.

2. The method of claim 1 further characterized in that the allograft is contacted with at least about 1 µg of immunotoxin per $1 \times 10^6$ Ia equivalent antigen-bearing cells of the allograft.

3. The method of claim 1 further characterized in that the allograft is a human allograft and the Ia equivalent antigen is an HLA-DR antigen.

4. The method of claim 1 further characterized in that the immunotoxin is a conjugate of an anti-Ia equivalent monoclonal antibody and ricin A chain.

5. An allograft characterized in that the allograft is pretreated with an immunotoxin to an Ia equivalent antigen borne by the allograft.

6. The allograft of claim 5 further characterized in that the allograft is a human allograft and the Ia equivalent antigen is an HLA-DR antigen.

7. The allograft of claim 5 further characterized in that the immunotoxin is a conjugate of an anti-Ia equivalent monoclonal antibody and ricin A chain.

8. An immunotoxin comprising a toxin component conjugated to an antibody wherein said antibody is directed to an Ia equivalent antigen.

9. The immunotoxin of claim 8 further characterized in that the Ia equivalent antigen is an HLA-DR antigen.

10. The immunotoxin of claim 8 further characterized in that the toxin component is a ricin A chain and the antibody is a monoclonal antibody.

**Patentansprüche**

1. Verfahren zur Reduzierung der Immunogenität eines Allotransplantats, dadurch gekennzeichnet, daß das Allotransplantat vor der Transplantation mit einem gegen ein Ia-äquivalentes Antigen des Allotransplantats gerichteten Immunotoxin in vitro in Kontakt gebracht wird.

2. Verfahren nach Anspruch 1, das ferner dadurch gekennzeichnet ist, daß das Allotransplantat mit mindestens etwa 1 μg Immunotoxin pro $1 \times 10^6$ Ia-äquivalentes Antigentragenden Zellen des Allotransplantats in Kontakt gebracht wird.

3. Verfahren nach Anspruch 1, das ferner dadurch gekennzeichnet ist, daß das Allotransplantat ein menschliches Allotransplantat und das Ia-äquivalente Antigen ein HLA-DR-Antigen ist.

4. Verfahren nach Anspruch 1, das ferner dadurch gekennzeichnet ist, daß das Immunotoxin ein Konjugat eines anti-Ia-äquivalenten monoclonalen Antikörpers und einer Ricin A-Kette ist.

5. Allotransplantat, dadurch gekennzeichnet, daß das Allotransplantat mit einem Immunotoxin gegen ein Ia-äquivalentes Antigen des Allotransplantats vorbehandelt ist.

6. Allotransplantat nach Anspruch 5, das ferner dadurch gekennzeichnet ist, daß das Allotransplantat ein menschliches Allotransplantat und das Ia-äquivalente Antigen ein HLA-DR-Antigen ist.

7. Allotransplantat nach Anspruch 5, das ferner dadurch gekennzeichnet ist, daß das Immunotoxin ein Konjugat aus einem anti-Ia-äquivalenten monoclonalen Antikörper und einer Ricin-A-Kette ist.

8. Immunotoxin, bei dem ein Toxin-Bestandteil mit einem Antikörper konjugiert ist, wobei der Antikörper gegen ein Ia-äquivalentes Antigen gerichtet ist.

9. Immunotoxin nach Anspruch 8, das ferner dadurch gekennzeichnet ist, daß das Ia-äquivalente Antigen ein HLA-DR-Antigen ist.

10. Immunotoxin nach Anspruch 8, das ferner dadurch gekennzeichnet ist, daß der Toxin-Bestandteil eine Ricin A-Kette und der Antikörper ein monoclonaler Antikörper ist.

**Revendications**

1. Procédé pour affaiblir l'immunogénicité d'une allogreffe, caractérisé en ce que l'allogreffe est mise en contact *in vitro*, avant la transplantation, avec une immunotoxine contre un antigène equivalent à la porté par l'allogreffe.

2. Procédé suivant la revendication 1, caractérisé, en outre, en ce que l'allogreffe est mise en contact avec au moins environ 1 μg d'immunotoxine pour $1 \times 10^6$ cellules porteuses de l'antigène équivalent à la de l'allogreffe.

3. Procédé suivant la revendication 1, caractérisé, en outre, en ce que l'allogreffe est une allogreffe humaine et l'antigène équivalent à la est un antigène HLA-DR.

4. Procédé suivant la revendication 1, caractérisé, en outre, en ce que l'immunotoxine est un conjugué d'un anticorps monoclonal anti-équivalent à la et de la chaîne A de ricine.

5. Allogreffe, caractérisée en ce que l'allogreffe est traitée au préalable avec une immunotoxine contre un antigène équivalent à la porté par l'allogreffe.

6. Allogreffe suivant la revendication 5, caractérisée, en outre, en ce que 'allogreffe est une allogreffe humaine et l'antigène équivalent à la est un antigène HLA-DR.

7. Allogreffe suivant la revendication 5, caractérisée, en outre, en ce que l'immunotoxine est un conjugué d'un anticorps monoclonal anti-équivalent à la et de la chaîne A de ricine.

8. Immunotoxine comprenant un composant toxine conjugué à un anticorps, dans laquelle l'anticorps est dirigé contre un antigène équivalent à la.

9. Immunotoxine suivant la revendication 8, caractérisée, en outre, en ce que l'antigène équivalent à I est un antigène HLA-DR.

10. Immunotoxine suivant la revendication 8, caractérisée en ce que le composant toxine est la chaîne A de ricine et l'anticorps est un anticorps monoclonal.

FIG .1.

FIG.2.